# EUROPÄISCHE PATENTANMELDUNG

(11) **EP 1 437 085 A1**
(43) Veröffentlichungstag der Anmeldung: **14.07.2004**
(21) Anmeldenummer: 03022462.0
(22) Anmeldetag: 08.10.2003
(51) Int. Cl.: A61B 3/103

(54) **Augenrefraktometer und Verfahren zum Betrieb eines Augenrefraktometers**

(30) Priorität: 09.01.2003 DE 10300322
(71) Anmelder: Oculus Optikgeräte GmbH, 35582 Wetzlar-Dutenhofen (DE)
(72) Erfinder: Köst, Gerd, 30171 Hannover (DE)
(74) Vertreter: Böck, Bernhard, Dipl.-Ing.

(57) **Zusammenfassung**

Die Erfindung betrifft ein Augenrefraktometer (13) zur objektiven Brechkraftbestimmung eines Auges (A), mit einem Optometersystem zur Abbildung einer Testmarke auf die Netzhaut des Auges (A) und mit einem Beobachtungssystem (15) zur Beobachtung der auf die Netzhaut abgebildeten Testmarke (18), wobei Optometersystem und Beobachtungssystem synchron zueinander relativ zu eine Referenzstellung verstellt werden können, und wobei aus der Differenz zwischen der Einstellung, bei der die Testmarke (18) zumindest teilweise konturscharf auf die Netzhaut abgebildet wird, und der Referenzstellung ein Brechkraftparameter des Auges (A) bestimmbar ist. Am Beobachtungssystem ist eine digitale Aufnahmeeinrichtig (42) und eine digitale Bildverarbeitungseinheit vorgesehen, wobei mit der Aufnahmeeinrichtig (42) digitale Bilddaten der auf die Netzhaut abgebildeten Testmarke (18) aufgenommen werden können, und wobei die dabei erhaltenen Bilddaten in der Bildverarbeitungseinheit durch digitale Bildverarbeitung zur Feststellung des Brechkraftparameters ausgewertet werden können.

## Beschreibung

Die Erfindung bezieht sich auf ein Augenrefraktometer und ein Verfahren zum Betrieb eines Augenrefraktometers nach dem Oberbegriff der unabhängigen Hauptansprüche.

Derartige Augenrefraktometer sind aus dem Stand der Technik bekannt und dienen der objektiven Brechkraftbestimmung des Auges. Die Augenrefraktometrie ist wesentliche Grundlage der Früherkennung von Augenfehlern sowie der Herstellung von Brillen.

Das menschliche Auge arbeitet durch die Erfassung und Fokussierung von Lichtstrahlen. Die Lichtstrahlen werden bei ihrem Durchlauf durch die Hornhaut, das Kammerwasser, die Linse und den Glaskörper fokussiert. Idealerweise liegt der Fokuspunkt des Lichtes nach dem Durchlaufen dieser Komponenten auf der Netzhaut, so dass auf der Netzhaut ein konturscharfes Bild der Umgebung abgebildet wird. Die Normalsichtigkeit oder das Nicht-Vorhandensein eines Brechungsfehlers ist somit durch den Fokuspunkt des Lichts gekennzeichnet, das aus unendlichem Abstand in das Auge eintritt und fokussiert auf die Netzhaut fällt.

Aufgrund von Brechungsfehlern des Auges leiden viele Menschen an Fehlsichtigkeit, die dadurch gekennzeichnet ist, dass der Fokuspunkt des Lichts nicht auf die Netzhaut, sondern auf einen Punkt kurz davor (Kurzsichtigkeit) oder auf einen Punkt kurz dahinter (Weitsichtigkeit) fällt. Ein solcher Fehler der Brechkraft erster Ordnung kann mit einer sphärisch ausgebildeten Linse korrigiert werden, so dass durch die Kombination der sphärischen Linse mit dem Linsensystem des Auges erreicht wird, dass das Licht dadurch auf die Netzhaut fokussiert wird.

Diesem Brechkraftfehler erster Ordnung kann auch ein Brechkraftfehler zweiter Ordnung, ein sogenannter Astigmatismus überlagert sein. Der Astigmatismus ergibt sich, wenn das Auge in unterschiedlichen Meridianen unterschiedliche Brechungsfehler aufweist. Einen solchen Brechkraftfehler zweiter Ordnung kann man durch die zusätzliche Kombination einer zylindrischen Linse zu der sphärischen Linse ausgleichen. Bei der Herstellung von zum Ausgleich der Brechkraftfehler erster und zweiter Ordnung geeigneter Brillengläser ist es deshalb üblich einen Parametersatz mit drei Parametern anzugeben, um die Brechkraftfehler erster und zweiter Ordnung eindeutig beschreiben. Der erste Brechkraftparameter D1 gibt die Brechkraft des Auges in Richtung der ersten Hauptachse an. Der zweite Brechkraftparameter D2 gibt die Brechkraft des Auges an der zweiten Hauptachse an. Die Brechkraftparameter D1 und D2 werden üblicherweise in Dioptrien angegeben. Der dritte Brechkraftparameter α gibt den Winkel der Hauptachsen relativ zur Vertikalen beziehungsweise zur Horizontalen an und entspricht letztendlich dem Winkel, unter dem die Längsachse der Zylinderlinse relativ zur Vertikalen beziehungsweise Horizontalen verläuft.

Mit den bekannten Augenrefraktometern können die verschiedenen Brechkraftparameter des Auges objektiv vermessen werden. Dazu ist bei jedem Augenrefraktometer ein Optometersystem zur Abbildung einer Testmarke auf die Netzhaut des Auges und ein Beobachtungssystem zur Beobachtung der auf die Netzhaut abgebildeten Testmarke vorhanden. Ein geeigneter Strahlenteiler sorgt dabei für eine Trennung des in das Auge einfallenden Strahlengangs (Optometersystem) vom aus dem Auge austretenden Strahlengang (Beobachtungssystem). Optometersystem und Beobachtungssystem können unter entsprechender Änderung ihrer optischen Eigenschaften synchron zueinander relativ zu einer bestimmten Referenzstellung verstellt werden. Die Referenzstellung entspricht dabei der Einstellung, bei der die Testmarke unter Verwendung eines Prüfkörpers, dessen optische Eigenschaften einem Idealauge ohne Brechkraftfehler entspricht, vom Optometersystem fokussiert abgebildet und durch das Beobachtungssystem konturscharf beobachtet werden kann. Wird dann ein Auge mit Brechkraftfehler durch den Augenrefraktometer betrachtet, so wird die Testmarke in der Referenzstellung des Augenrefraktometers unscharf auf der Netzhaut abgebildet und entsprechend unscharf durch das Beobachtungssystem beobachtet.

Bei der Messung wird dann des Optometersystem und das Beobachtungssystem so lange synchron verstellt, bis die Testmarke auf der Netzhaut fokussiert abgebildet und konturscharf durch das Beobachtungssystem beobachtet werden kann. Aus der Differenz dieser Einstellung zur Referenzstellung kann dann ein Brechkraftparameter bestimmt werden.

Sind an dem Auge auch Brechkraftfehler höherer Ordnung vorhanden, so wird die Testmarke in verschiedenen Stellungen, deren Anzahl der Ordnungszahl des Brechkraftfehlers entspricht, jeweils teilweise konturscharf auf der Netzhaut abgebildet. Aus dieser Erkenntnis heraus können auch Brechkraftfehler höherer Ordnung durch entsprechende Einstellung des Augenrefraktometers ermittelt werden.

Zur Messung des Winkels α, der die Stellung der Hauptachsen relativ zur Horizontalen beziehungsweise Vertikalen angibt, kann beispielsweise eine sogenannte "Raubitscheck-Kurve" als Testmarke verwendet werden. Das Verschwenken dieser "Raubitscheck-Kurve" in der Stellung, in der die Testmarke das erste Mal auf der Netzhaut fokussiert wird, erlaubt die Bestimmung des Winkels α.

Aus der US 4,410,243 und der DE 30 14 907 A1 sind jeweils Augenrefraktometer bekannt, bei denen im Beobachtungssystem ein beziehungsweise zwei Okulare vorgesehen sind, durch die hindurch der Behandler die auf die Netzhaut abgebildete Testmarke beobachten kann. Nachteilig an diesen Systemen ist es, dass die Messung der Brechkraftfehler für den Behandler relativ anstrengend ist und außerdem die Messgenauigkeit von der Sorgfalt des jeweiligen Behandlers abhängt.

Aus der DE 30 37 481 C2 und der DE 31 02 450 C2 sind jeweils Augenrefraktometer bekannt, bei denen im Beobachtungssystem Einrichtungen zur elektronischen Messsignalverarbeitung vorgesehen sind. Dadurch wird es durch entsprechende Auswertung der elektronischen Messsignale möglich, die Einstellungen des Augenrefraktometers festzustellen, in denen die Testmarke auf der Netzhaut fokussiert ist. Nachteilig an diesen Geräten ist es, dass sie einen hohen gerätetechnischen Aufwand erfordern und vielfach nur eine unzureichende Messqualität bieten.

Aufgabe der vorliegenden Erfindung ist es deshalb, ein neues Augenrefraktometer vorzuschlagen, das die Nachteile des bekannten Stands der Technik vermeidet. Weiter ist es Aufgabe der vorliegenden Erfindung ein Verfahren zur Benutzung eines Augenrefraktometers anzugeben, mit dem in einfacher Weise sehr gute Messergebnisse erreicht werden können.

Diese Aufgabe wird durch das Augenrefraktometer beziehungsweise das Verfahren zur Benutzung eines Augenrefraktometers gemäß der Lehre der beiden unabhängigen Hauptansprüche gelöst.

Vorteilhafte Ausführungsformen sind Gegenstand der Unteransprüche.

Erfindungsgemäß ist am Beobachtungssystem des Augenrefraktometers eine digitale Aufnahmeeinrichtung vorgesehen, die mit einer Bildverarbeitungseinheit verbunden ist. Die Bildverarbeitungseinheit kann beispielsweise in der Art eines Standardrechners ausgebildet sein, auf dem eine entsprechend geeignete Software installiert ist. Alternativ dazu sind auch hardwaremäßig realisierte Bildverarbeitungseinheiten denkbar. Entsprechend der normalen Funktion des Augenrefraktometers wird die Testmarke auf die Netzhaut des Auges abgebildet und mittels der digitalen Aufnahmeeinrichtung aufgenommen. Die so erhaltenen digitalen Bilddaten der auf die Netzhaut abgebildeten Testmarke werden in der Bildverarbeitungseinheit ausgewertet um einen oder mehrere Brechkraftparameter des Auges festzustellen.

Nach einer bevorzugten Ausführungsform der Erfindung ist die digitale Aufnahmeeinrichtung in der Art eines CCD-Chips ausgebildet. Derartige CCD-Chips weisen ein ausreichend hohes Auflösungsvermögen auf und sind kostengünstig verfügbar.

In welcher Weise die synchrone Verstellung von Optometersystem und Beobachtungssystem erfolgt, ist grundsätzlich beliebig. Aus dem Stand der Technik sind verschiedenste Möglichkeiten zur Verstellung von Optometersystem und Beobachtungssystem und der Synchronisation der beiden Stellbewegungen bekannt. Ein besonders einfacher konstruktiver Aufbau des Augenrefraktometers lässt sich erzielen, wenn am Augenrefraktometer eine linear verstellbare Stelleinheit vorgesehen ist, auf der ein erstes und ein zweites Umlenkelement befestigt sind. Das erst Umlenkelement ist dabei im Strahlengang des Optometersystems angeordnet, wohingegen das zweite Umlenkelement im Strahlengang des Beobachtungssystems angeordnet ist. Die beiden Umlenkelemente sind dabei derart gestaltet und angeordnet, dass durch die lineare Verstellung der Stelleinheit die Länge des Strahlenganges im Optometersystem und im Beobachtungssystem verändert wird. Da beide Umlenkelemente in gleicher Weise fest mit der Stelleinheit verbunden sind, wird durch die einfache lineare Verstellung der Stelleinheit eine synchrone Längenänderung im Strahlengang des Optometersystems und des Beobachtungssystems realisiert.

Zur konstruktiven Realisierung der beiden Umlenkelemente sind eine Vielzahl von Ausführungsformen denkbar. Nach einer bevorzugten Ausführungsform werden die Umlenkelemente jeweils von zwei Reflektionselementen, beispielsweise Spiegeln oder Spiegelprismen gebildet, die im Strahlengang des Optometersystems beziehungsweise des Beobachtungssystems angeordnet sind. Die beiden Reflexionselemente bilden dabei im jeweiligen Strahlengang einen Posaunenzug, das heißt, der Lichtstrahl wird an den Reflexionselementen um zwei Mal 90° abgelenkt, wobei der Abstand zwischen dem am ersten Reflexionselement einfallenden Lichtstrahl und dem am zweiten Reflexionselement abgestrahlten Lichtstrahl konstant bleibt. Durch eine solche posaunen-zugartige Umlenkung des Lichts im Strahlengang des Optometersystems beziehungsweise Beobachtungssystems ist es in einfacher Weise möglich, durch lineare Verstellung der auf der Stelleinheit paarweise angeordnete Reflexionselemente die Länge des jeweiligen Strahlengangs zu verändern.

Um die Anforderungen an die Fertigungstoleranzen absenken zu können, ist es besonders vorteilhaft, wenn die Umlenkelemente auf der Stelleinheit justierbar gelagert sind. Dadurch wird es möglich, bei der Montage des Augenrefraktometers die Umlenkelemente exakt auf der Stelleinheit zu justieren, um genaue Messungen zu ermöglichen. Auch bei der Feststellung von späteren Messfehlern kann durch entsprechende Justierung der Umlenkelemente Abhilfe geschaffen werden.

Von entscheidender Bedeutung für die exakte Messung der Brechkraft des Auges ist es, dass die Länge des Strahlengangs im Beobachtungssystem exakt mit der Länge des Strahlengangs im Optometersystem übereinstimmt. Um diese exakte Übereinstimmung der Länge der beiden Strahlengänge auch bei entsprechend unterschiedlichen Strahlengangführungen realisieren zu können, kann zwischen dem ersten Umlenkelement und dem zweiten Umlenkelement ein Versatz in der Stellrichtung vorhanden sein. Dieser Versatz bewirkt einen fest voreingestellten Offset, durch den Unterschiede in der Länge anderer Strahlengangabschnitte ausgeglichen werden können.

In welcher Weise die Stelleinheit zur Verstellung angetrieben wird, ist grundsätzlich beliebig. Nach einer bevorzugten Ausführungsform wird die Stelleinheit jedoch mit einem elektronischen Servomotor angetrieben. An derartigen Servomotoren kann die jeweilige Ist-Stellung sensorisch abgegriffen und als entsprechender Einstellungswert des Augenrefraktometers an die Auswerteeinheit weitergeleitet werden. Außerdem können solche Servomotoren lagegeregelt werden, damit die Stelleinheit exakt vorgegebene Positionen anfahren kann, in denen dann jeweils eine digitale Bildaufnahme der auf die Netzhaut abgebildeten Testmarke gemacht wird. Der Servomotor kann dabei in der Art eines Schrittmotors betrieben werden können, so dass die Verstellung der Stelleinheit in äquidistanten Schritten entlang des Stellweges erfolgen kann. An den äquidistant voneinander entfernten Haltepunkten der Stelleinheit wird dann jeweils eine digitale Bildaufnahme gemacht, die nachfolgend in der Auswerteeinheit ausgewertet werden kann.

Zur Feststellung des Hauptachsenwinkels α kann beispielsweise die sogenannte "Raubitscheck-Kurve" verwendet werden. Diese spezielle Testmarke erfordert jedoch die Verstellung durch einen erfahrenen Behandler, was relativ zeitaufwändig ist. Es wird deshalb vorgeschlagen, als Testmarke ein mittelpunktsymmetrisches Konturbild mit mehreren sich vom Mittelpunkt nach außen erstreckenden Konturübergängen zu verwenden. Dabei sollte die Testmarke vorzugsweise eine Vielzahl von abwechselnd angeordneten Hell- und Dunkelfeldern aufweisen. Insbesondere Testmarken, deren Gestalt einem "Siemensstern" entsprechen, sind zur Verwendung mit dem erfindungsgemäßen Augenrefraktometer geeignet.

Das erfindungsgemäße Verfahren zum Betrieb eines Augenrefraktometers zeichnet sich dadurch aus, dass zunächst bei unterschiedlichen Einstellungen des Optometersystems beziehungsweise des Beobachtungssystems mit der digitalen Aufnahmeeinrichtung mehrere digitale Bilddatensätze aufgenommen und zusammen mit den jeweils zugeordneten Einstellparametern gespeichert werden. Ist zur Verstellung des Optometersystems beziehungsweise des Beobachtungssystems beispielsweise eine linear verstellbare Stelleinheit vorgesehen, so wird diese Stelleinheit entlang dem Stellweg verfahren, wobei an bestimmten Haltepunkten jeweils eine Aufnahme der auf die Netzhaut abgebildeten Testmarke gemacht wird. Jede dieser digitalen Aufnahmen bildet einen Bilddatensatz und wird zusammen mit der jeweiligen Einstellung gespeichert, das heißt im Beispiel mit dem entsprechenden linearen Stellwert.

Die so erhaltenen Bilddatensätze werden erfindungsgemäß in einer Bildverarbeitungseinheit durch digitale Bildverarbeitung ausgewertet. Die Bildverarbeitung kann dabei nach einer ersten Ausführungsform in Echtzeit erfolgen, so dass die Bilddatensätze parallel zur Aufnahme durch die digitale Aufnahmeeinrichtung in der Bildverarbeitungseinheit ausgewertet werden. Eine solche Echtzeitverarbeitung erfordert jedoch eine sehr hohe Rechenleistung. Steht eine solche Rechenleistung nicht zur Verfügung, werden die Bilddatensätze zunächst abgespeichert und erst nach Abschluss der Aufnahme von allen Bilddatensätzen ausgewertet. Dies ermöglicht es, dass die Aufnahmen sehr schnell gemacht werden, so dass der Behandelte seine Augen nur relativ kurz vor dem Augenrefraktometer positionieren muss.

Durch Anwendung entsprechend geeigneter Berechnungsmethoden wird in der Bildverarbeitungseinheit jedem Bilddatensatz zumindest eine Konturschärfenbewertung zugeordnet. Dies bedeutet mit anderen Worten, dass durch die Bildverarbeitungseinheit die konturscharfe Abbildung der Testmarke auf die Netzhaut bewertet und mit einem oder mehreren Parametern klassifiziert wird.

Liegen für alle Bilddatensätze die Parameter zur Konturschärfenbewertung vor, so werden diese in einer Auswerteeinheit, die beispielsweise durch eine auf einem Standartrechner installierte Software realisiert sein kann, ausgewertet. Durch die Auswerteeinheit wird dann festgestellt, für welche Einstellungen des Augenrefraktometers es relative Maximalwerte für die Konturschärfenbewertung gibt. Diese relativen Maxima der Konturschärfenbewertung sind nämlich für die Brechkraft des Auges charakteristisch, so dass in der Auswerteeinheit aus der Differenz zwischen den Einstellungen, in denen es relative Maximalwerte für die Konturschärfenbewertung gibt, und der Referenzstellung Brechkraftparameter des untersuchten Auges abgeleitet werden können.

In der Augenheilkunde werden üblicherweise nur Brechkraftfehler erster und zweiter Ordnung berücksichtigt. Es ist deshalb besonders vorteilhaft, wenn in der Auswerteeinheit die Bilddatensätze mit den beiden höchsten relativen Maximalwerten festgestellt werden. Aus den Einstellungen des Augenrefraktometers, die diesen relativen Maximalwerten zugeordnet sind, lassen sich die Brechkraftparameter in den beiden Brechkrafthauptachsen ableiten, so dass die Angabe der üblichen Parameter zur Herstellung der für die Korrektur des Brechkraftfehlers notwendigen sphärozylindrischen Linse schnell umrechenbar ist.

Soll neben den beiden Dioptrienwerten für die Brechkraftkorrektur in den beiden Hauptachsen auch noch der Winkel α ermittelt werden, so kann eine vorzugswürdige Verfahrensvariante angewendet werden. Bei dieser Verfahrensvariante wird als Testmarke ein mittelpunktsymmetrisches Konturbild mit mehreren sich von vom Mittelpunkt nach außen erstreckenden Konturübergängen verwendet. Weist das untersuchte Auge einen Brechkraftfehler zweiter Ordnung auf, so führt dies dazu, dass dieses mittelpunktsymmetrische Konturbild bei keiner Einstellung des Augenrefraktometers vollständig fokussiert abgebildet wird. Vielmehr sind auch die Bilddatensätze mit den relativen Maximalwerten für die Konturschärfebewertung nur bereichsweise konturscharf sind. Andere Bereiche werden dagegen auch bei diesen Bilddatensätzen mit relativen Maximalwerten für die Konturschärfenbewertung nur leicht verschwommen abgebildet. Um den Winkel α abzuleiten, wird nun in den Bilddatensätzen mit relativen Maximalwerten für die Konturschärfebewertung der jeweilige Konturübergang gesucht, an dem die Testmarke mit maximaler Konturschärfe abgebildet wurde. Aus der Winkeldifferenz γ zwischen diesem Konturübergang mit maximaler Konturschärfe und der Vertikalen beziehungsweise Horizontalen wird dann der Winkel α abgeleitet.

Für die bei Durchführung des Verfahrens erforderliche Konturschärfenbewertung der Bilddatensätze können verschiedene Berechnungsalgorithmen eingesetzt werden.

Ist an dem Augenrefraktor eine Fixationsbeleuchtung zur Fixation des Auges in einer Lage vorgesehen ist, in der mit dem Beobachtungssystem der Fundus des Auges, insbesondere der Nervenfaserkopf, aufgenommen werden kann, so kann der Augenrefraktor ohne größere zusätzliche Maßnahmen als Funduskamera benutzt werden. Zur unstrukturierten Beleuchtung des Fundus muss lediglich eine entsprechend geeignete Beleuchtungseinrichtung vorgesehen sein. Durch entsprechende Auswertung der Bilddaten kann insbesondere auch die Tiefe der Exkavitation des Nervenfaserkopfs bestimmt werden.

Nachfolgend wird die Erfindung anhand der Zeichnungen beispielhaft erläutert.

Es zeigen:
- **Fig. 1:**: ein normalsichtiges Auge im schematischen Querschnitt;
- **Fig. 2:**: ein kurzsichtiges Auge im schematischen Querschnitt;
- **Fig. 3:**: ein weitsichtiges Auge im schematischen Querschnitt;
- **Fig. 4:**: eine sphäro-zylindrische Korrekturlinse zur Korrektur von Brechkraftfehlern erster und zweiter Ordnung in schematischer Ansicht von vorne;
- **Fig. 5:**: ein Augenrefraktometer in schematischer Ansicht von oben;
- **Fig. 6:**: ein schematisiertes Messdiagramm der Konturschärfenbewertung verschiedener Aufnahmen der Testmarke angetragen über den Stellweg des Augenrefraktometers;
- **Fig. 7:**: das Konturbild einer zur Abbildung auf die Netzhaut des Auges geeigneten Testmarke;
- **Fig. 8:**: eine schematisierte Bildaufnahme der Abbildung der in Fig. 7 dargestellten Testmarke auf die Netzhaut.

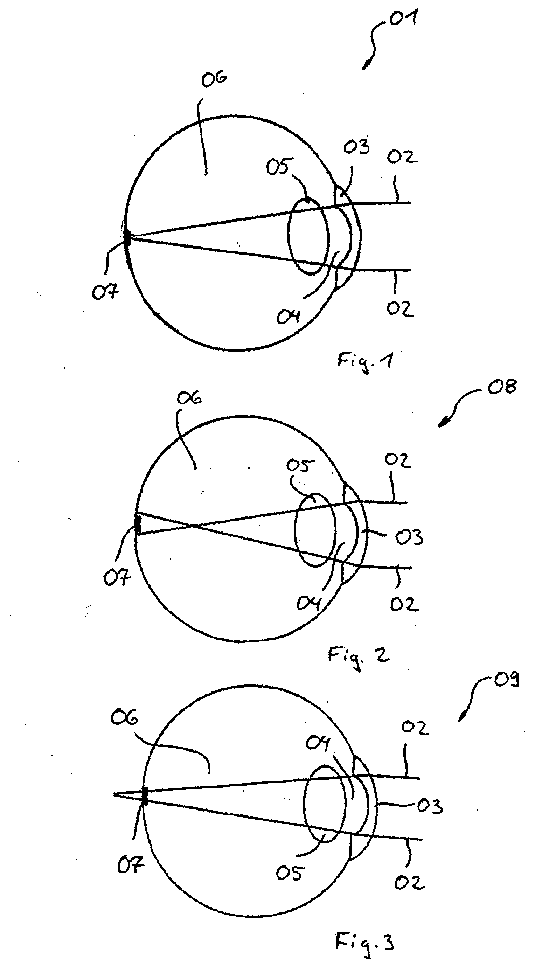

**Fig. 1** stellt den Zustand eines schematisch im Querschnitt dargestellten Auges 01 bei Normalsichtigkeit dar. Wie in **Fig. 1** dargestellt, treten die Lichtstrahlen 02 parallel verlaufend aus dem Unendlichen kommend über die Hornhaut 03 in das Auge 01 ein, verlaufen durch die Hornhaut 03, das Kammerwasser 04, die Linse 05 und den Glaskörper 06. Die Lichtstrahlen 02 werden durch das Brechungsvermögen der Hornhaut 03, des Kammerwassers 04, der Linse 05 und des Glaskörpers 06 auf einen Fokuspunkt fokussiert, der im Falle der Normalsichtigkeit, wie in **Fig. 1** dargestellt, auf der Netzhaut 07 des Auges 01 liegt.

Ist ein Auge 08, wie in **Fig. 2** dargestellt, dagegen kurzsichtig, so werden die aus dem Unendlichen parallel einfallenden Lichtstrahlen 02 nicht auf der Netzhaut 07, sondern kurz davor fokussiert, so dass auf der Netzhaut 07 nur eine unscharfe Abbildung entsteht.

Ist ein Auge 09 dagegen, wie in **Fig. 3** dargestellt, weitsichtig, so erfolgt die Fokussierung der aus dem Unendlichen parallel einfallenden Lichtstrahlen 02 kurz hinter der Netzhaut 07, was wiederum zu einer verschwommenen Abbildung auf der Netzhaut 07 führt.

Die in **Fig. 2** und **Fig. 3** dargestellten Fehlsichtigkeiten werden als Brechkraftfehler erster Ordnung bezeichnet und können durch Tragen von sphärisch ausgebildeten Brillenlinsen korrigiert werden. Die entsprechende sphärische Brillenlinse ist dabei so zu gestalten, dass die Fokussierung der aus dem Unendlichen parallel einfallenden Lichtstrahlen jeweils auf der Netzhaut 07 erfolgt.

Neben den in **Fig. 2** und **Fig. 3** dargestellten Brechkraftfehlern erster Ordnung können am Auge auch noch Brechkraftfehler zweiter Ordnung auftreten. Derartige Brechkraftfehler zweiter Ordnung bedeuten, dass die Brechkraft des Auges nicht überall gleich ist, sondern vom Winkel relativ zur Vertikalen beziehungsweise Horizontalen abhängt. Derartige Brechkraftfehler zweiter Ordnung können durch eine zylindrische Korrekturlinse ausgeglichen werden. Bei üblichen Sehhilfen, wie Brillen oder Kontaktlinsen, werden deshalb zu deren Herstellung drei Brechkraftparameter angegeben. Der erste Brechkraftparameter D1 gibt die Brechkraft der sphärischen Korrekturlinse an. Der Brechkraftparameter D2 gibt die Brechkraft der zylindrischen Korrekturlinse an. Die Angabe der Brechkraft erfolgt dabei in Dioptrien. Der dritte Brechkraftparameter gibt den Winkel α an, unter dem sich die Mittelachse der zylindrischen Korrekturlinse relativ zur Horizontalen beziehungsweise zur Vertikalen erstreckt.

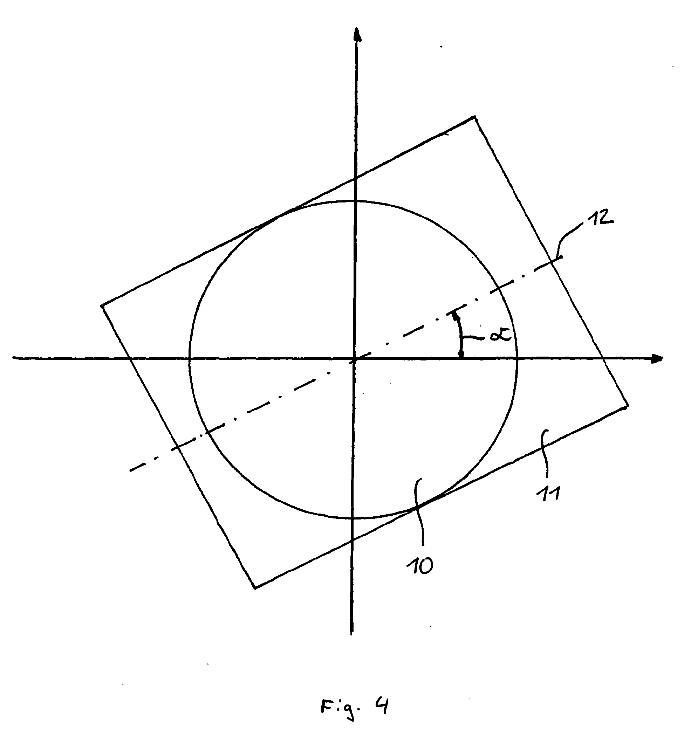

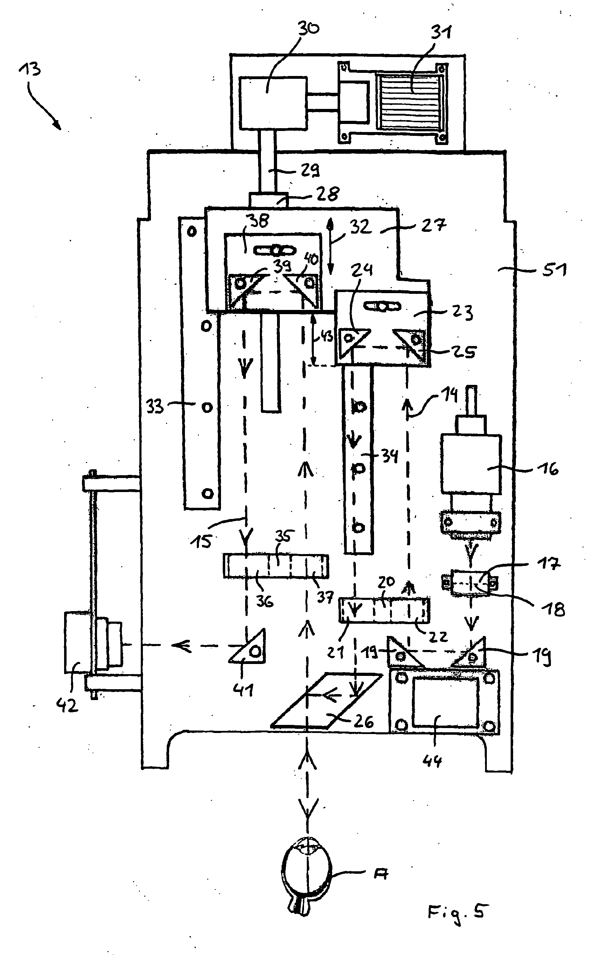

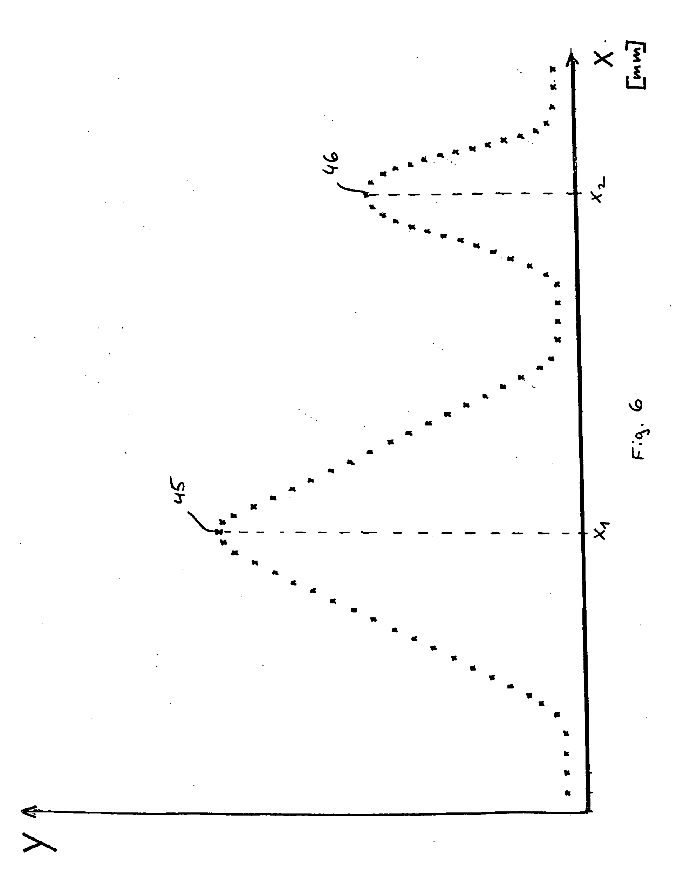

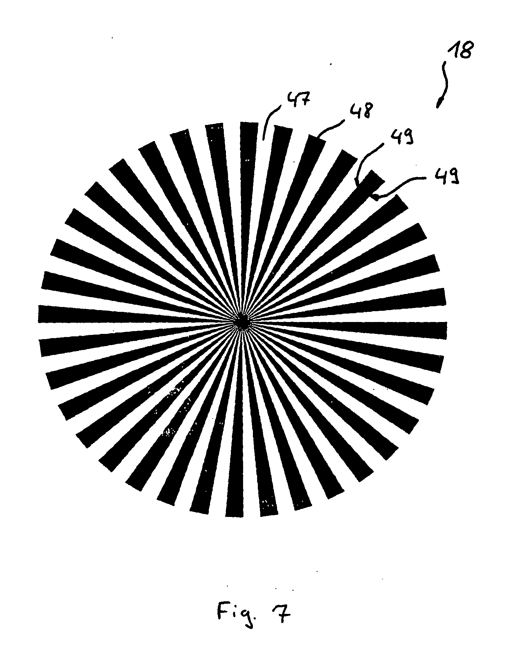

In **Fig. 4** ist eine schematisch dargestellte sphäro-zylindrische Korrekturlinse in Ansicht von vorne dargestellt. Man erkennt, dass die Korrekturlinse aus einer sphärischen Korrekturlinse 10 und einer davor beziehungsweise dahinter angeordneten zylindrischen Korrekturlinse 11 kombiniert ist. Zur Beschreibung der in **Fig. 4** dargestellten Korrekturlinse muss die Brechkraft der sphärischen Korrekturlinse 10, die Brechkraft der zylindrischen Korrekturlinse 11 und der Winkel α zwischen der Mittelachse 12 der zylindrischen Korrekturlinse und der Horizontalen (beziehungsweise Vertikalen) angegeben werden.

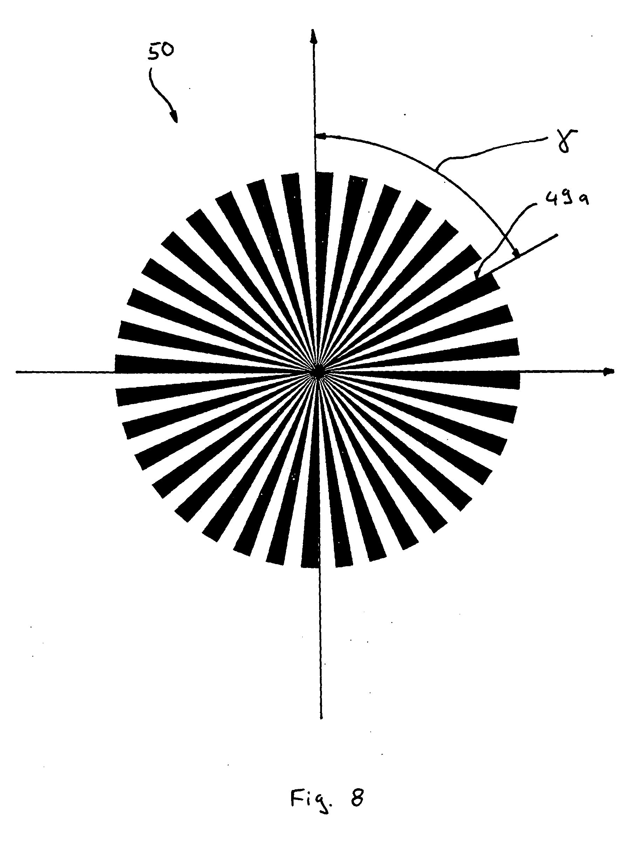

In **Fig. 5** ist ein erfindungsgemäßes Augenrefraktometer 13 in schematischer Ansicht von oben dargestellt. Das Augenrefraktometer 13 weist ein Optometersystem auf, mit dem ein Strahlengang 14 auf die Netzhaut eines zu untersuchenden Auges 15 gerichtet werden kann. Weiter weist das Augenrefraktometer 13 ein Beobachtungssystem auf, so dass über einen Strahlengang 15 die Netzhaut des untersuchten Auges beobachtet werden kann.

Das Optometersystem des Augenrefraktometers 13 wird im Wesentlichen von einer Lichtquelle 16, die beispielsweise in der Art einer LED-Beleuchtungseinrichtung ausgebildet sein kann, einem Testmarkenhalter 17 mit darin befestigter Testmarke 18 (siehe **Fig. 7**), zwei feststehend montierten Reflektorelementen 19, einem Trägerelement 20 mit zwei darin befestigten Linsen 21 und 22, einem ersten Umlenkelement 23 mit zwei darauf befestigten Reflexionselementen 24 und 25 und einem Spiegelprisma 26 gebildet. Die Lichtquelle 16, der Testmarkenhalter 17, die Reflektorelemente 19, das Trägerelement 20 mit den Linsen 21 und 22 und das Spiegelprisma 26 sind feststehend auf einer Grundplatte 51 montiert. Das Umlenkelement 23 mit den Reflexionselementen 24 und 25 dagegen ist justierbar auf einer in der Art eines Schlittens ausgebildeten Stelleinheit 27 befestigt. Die Stelleinheit 27 steht über eine Spindelmutter 28, eine Antriebsspindel 29 und ein Getriebe 30 mit einem Servomotor 31 im Eingriff, so dass durch Antrieb des Servomotors 31 die Stelleinheit 27 in Richtung des Bewegungspfeils 32 verstellt werden kann. Dazu ist die Stelleinheit 27 auf zwei Schienen 33 und 34, die justierbar auf der Grundplatte 51 befestigt sind, linear verstellbar gelagert.

Wie man aus dem Strahlengang 14 des Optometersystems erkennt, tritt das von der Lichtquelle 16 abgestrahlte Licht durch den Testmarkenhalter 17, so dass ein Konturbild der dort angebrachten Testmarke 18 entsteht. Durch Reflexion an den Reflektorelementen 19, 24, 25 und an den Reflexionsflächen des Spiegelprismas 26 wird der Lichtstrahl auf die Netzhaut des Auges 15 gelenkt, so dass dort das Konturbild der Testmarke 18 abgebildet wird. Die Länge des Strahlengangs 14 kann durch Verstellung der Stelleinheit 27 verlängert beziehungsweise verkürzt werden. Die Anordnung der Reflexionselemente 24 und 25 des ersten Umlenkelements 23 relativ zum linken Reflektorelement 19 und zum Spiegelprisma 26 bilden dazu einen sogenannten Posaunenzug. Eine Verstellung der Stelleinheit 27 um eine Maßeinheit bewirkt deshalb eine Verkürzung beziehungsweise Verlängerung des Strahlengangs 14 um jeweils genau zwei Maßeinheiten.

Das Beobachtungssystem des Augenrefraktometers 13 wird von einem Trägerelement 35 mit zwei darin befestigten Linsen 36 und 37, einem zweiten Umlenkelement 38 mit zwei darauf befestigten Reflexionselementen 39 und 40, einem Reflexionselement 41 und einer digitalen Aufnahmeeinrichtung 42, die in der Art eines CCD-Chips ausgebildet ist, gebildet.

Wie man aus dem Strahlengang 15 des Beobachtungssystems erkennen kann, wird das auf die Netzhaut des Auges A abgebildete Konturbild an der Netzhaut reflektiert und kann durch Reflexion an den Reflexionselementen 39, 40 und 41 mittels der Aufnahmeeinrichtung 42 beobachtet werden. Das Trägerelement 35 mit den Linsen 36 und 37 und das Reflexionselement 41 sind wiederum feststehend auf der Grundplatte 51 montiert. Das zweite Umlenkelement 38 mit den Reflexionselementen 39 und 14 ist entsprechend dem ersten Umlenkelement 23 justierbar auf der Stelleinheit 27 befestigt und kann somit durch Antrieb des Servomotors 31 synchron zum ersten Umlenkelement 23 verstellt werden. Dadurch, dass beide Umlenkelemente 23 und 38 gemeinsam auf der Stelleinheit 27 befestigt sind und jeweils einen posaunenzugartigen Strahlengangabschnitt bilden, wird die Synchronisation der Einstellung des Optometersystems und des Beobachtungssystems außerordentlich vereinfacht, da eine mechanische Koppelung der beiden Systeme zur synchronen Übertragung der Stellbewegung entfällt. Da der Strahlengang 14 und der Strahlengang 15 exakt gleich lang sein muss, sind die Umlenkelemente 23 und 38 mit einem Versatz 43 auf der Stelleinheit 27 befestigt. Durch den Versatz 43 werden die Lauflängenunterschiede der Strahlengänge 14 und 15, die sich aus der unterschiedlichen Anordnung der verschiedenen Reflexionselemente ergeben, ausgeglichen.

Da die Strahlengänge 14 und 15 jeweils zwei Linsen 21 und 22 beziehungsweise 36 und 37 durchlaufen, bewirkt eine Verstellung der Stelleinheit 27 und die damit verbundene Verkürzung beziehungsweise Verlängerung der Strahlengänge 14 und 15 eine Veränderung der Fokussierung im Optometersystem und im Beobachtungssystem. Diese Verstellung der Fokussierung erfolgt dabei jeweils synchron zueinander, da aufgrund der gemeinsamen Anordnung der Umlenkelemente 23 und 38 auf der Stelleinheit 27 die Änderung der Lauflänge in den Strahlengängen 14 und 15 jeweils exakt gleich ist.

Vor Einsatz des Augenrefraktometers 13 muss dieses zunächst kalibriert werden. Dazu wird anstelle des Auges A ein Testkörper eingesetzt, dessen optische Eigenschaften einem Idealauge ohne Brechkraftfehler entspricht. Anschließen wird die Stelleinheit 27 entlang des Stellweges so lange verstellt, bis von der Aufnahmeeinrichtung 42 ein konturscharfes Bild der abgebildeten Testmarke 18 aufgenommen wird. Die entsprechende Stellung der Stelleinheit 27 wird als Referenzstellung beziehungsweise Nullstellung gespeichert.

Zur Messung der Brechkraftparameter eines Auges A wird dann wie folgt vorgegangen:

Zunächst wird die Stelleinheit 27 durch Antrieb des Servomotors 31 bis zur hinteren Begrenzung des Stellbereichs verfahren, was der in **Fig. 5** dargestellten Position entspricht. Danach wird das Auge A vom Behandler relativ zur Grundplatte 51 des Augenrefraktometers 13 ausgerichtet. Dazu benutzt der Behandler eine Einrichtung 44, durch die eine direkte Beobachtung des Auges 15 durch Umlenkung am Spiegelprisma 26 ermöglicht wird. Anschließend wird mittels der Einrichtung 44 von oben eine Fixiermarke über das Spiegelprisma 26 ins Auge A eingeblendet, um die Pupille des Auges in einer vorgegebenen Achse zu fixieren.

Nachdem das Auge A auf diese Weise optimal ausgerichtet worden ist, wird die Lichtquelle 16 aktiviert um die Testmarke 18 auf der Netzhaut des Auges 15 über den Strahlengang 14 abzubilden. Diese Abbildung auf der Netzhaut wird durch den Strahlengang 15 von der Aufnahmeeinrichtung 42 beobachtet, so dass mit der Aufnahmeeinrichtung 42 ein digitales Bild angefertigt werden und als erster Bilddatensatz gespeichert werden kann. Anschließend wird die Stelleinheit 27 um einen bestimmten Betrag in Richtung des Bewegungspfeils 32 nach vorne verfahren und in einem bestimmten Abstand zur Startposition erneut angehalten. Mit dieser neuen Fokussierung der Strahlengänge 14 und 15 wird erneut eine digitale Bildaufnahme mit der Aufnahmeeinrichtung 42 aufgenommen und als weiterer Bilddatensatz gespeichert. In dieser Weise wird die Stelleinheit dann anschließend in einer Vielzahl von äquidistanten Schritten nach vorne gefahren, wobei an jedem einzelnen Haltepunkt mit der Aufnahmeeinrichtung 42 ein digitales Bild der auf die Netzhaut abgebildeten Testmarke 18 aufgenommen und gespeichert wird. Bei der Speicherung der Bilddatensätze werden außerdem jedem Bilddatensatz auch noch die Stellung der Stelleinheit 27 beziehungsweise des Servomotors 31 relativ zur Referenz- oder Nullstellung beigegeben und entsprechend zugeordnet abgespeichert. Sobald die Stelleinheit 27 das vordere Ende des Stellbereichs erreicht hat, kann der Behandelte das Auge A wieder aus der fixierten Stellung entfernen. Nach Abschluss dieses Aufnahmezyklussees steht ein Datenbestand zur Verfügung, der aus einer Vielzahl von digitalen Bildaufnahmen der auf die Netzhaut abgebildeten Testmarke 17 besteht, wobei jeder Bildaufnahme eine bestimmte Stellung der Stelleinheit 27 relativ zur Referenz- oder Nullstellung zugeordnet ist.

Im nächsten Schritt werden die einzelnen Bilddatensätze durch eine Bildverarbeitungseinheit, die beispielsweise durch Installation einer geeigneten Software auf einem Standartrechner realisiert sein kann, analysiert. Durch entsprechend geeignete Analysealgorithmen werden dabei jedem Bilddatensatz zumindest ein Wert Y zugeordnet. Durch Y wird dabei die Konturschärfe des Bildes klassifiziert, das durch den Bilddatensatz repräsentiert wird.

In Fig. 6 ist ein schematisches Diagramm dargestellt, in dem die Konturschärfebewertung Y der verschiedenen in einem Messzyklus Bilddatensätze über den Stellbereich der Stelleinheit 27 angetragen sind. Man erkennt, dass die Werte für die Konturschärfebewertung im Stellbereich der Stelleinheit 27 zwei relative Maximalwerte 45 und 46 annimmt. Aus den zugeordneten Stellwerten X1 und X2 der Stelleinheit 27 relativ zu der beim kalibrieren festgestellten Referenzstellung können die beiden Brechkraftparameter D1 und D2, durch die die Brechkraft der sphärozylindrischen Korrekturlinse bestimmt ist, abgeleitet werden.

Um auch den Winkel α aus den Messungen mit dem Augenrefraktometer 13 ableiten zu können, wird die in **Fig. 8** dargestellte Testmarke verwendet. Die Testmarke 18 ist in der Art eines Siemens-Sterns mit einer Vielzahl von abwechselnd angeordneten Hell- und Dunkelfeldern 47 und 48 ausgebildet. Die Hell- und Dunkelfelder 47 und 48 sind kreissegmentförmig ausgebildet, so dass im Ergebnis eine mittelpunktsymmetrische Kontur mit sich nach außen erstreckenden Konturübergängen 49 entsteht.

Die in **Fig. 8** dargestellte digitale Bildaufnahme 50 zeigt die Aufnahme der Testmarke 18, wie sie an der Position X2, dass heißt, beim niedrigeren relativen Maximalwert 46, aufgenommen wurde. Die Darstellung in **Fig. 8** ist dabei lediglich beispielhaft zu verstehen, da bei einer realen Bildaufnahme durch die Brechkraftfehler zweiter Ordnung des Auges A nur Teilbereiche der Testmarke 18 konturscharf abgebildet werden, wohingegen andere Bereiche leicht verschwimmen.

Durch geeignete Bildverarbeitungsalgorithmen wird in der digitalen Bildaufnahme 50 der Konturübergang 49 a festgestellt, der die relativ höchste Konturschärfe im Bild 50 aufweist. Aus dem Winkel γ zwischen der Vertikalen beziehungsweise Horizontalen kann dann durch geeignete Umrechnung ein Brechkraftparameter, nämlich Winkel α, abgeleitet werden.

### Bezugszeichenliste

- 01: Auge (normalsichtig)
- 02: Lichtstrahl
- 03: Hornhaut
- 04: Kammerwasser
- 05: Linse
- 06: Glaskörper
- 07: Netzhaut
- 08: Auge (kurzsichtig)
- 09: Auge (weitsichtig)
- 10: Sphärische Korrekturlinse
- 11: Zylindrische Korrekturlinse

- 12: Mittelachse (Zylindrische Korrekturlinse)

- 13: Augenrefraktometer

- 14: Strahlengang Optometersystem

- 15: Strahlengang
Beobachtungssystem
- 16: Lichtquelle (LED)
- 17: Testmarkenhalter
- 18: Testmarke
- 19: Reflektorelement
- 20: Trägerelement
- 21: Linse
- 22: Linse
- 23: Erstes Umlenkelement
- 24: Reflektorelement
- 25: Reflektorelement
- 26: Spiegelprisma
- 27: Stelleinheit
- 28: Spindelmutter
- 29: Antriebsspindel
- 30: Getriebe
- 31: Servomotor
- 32: Stellrichtung
- 33: Schiene
- 34: Schiene
- 35: Trägerelement
- 36: Linse
- 37: Linse
- 38: Zweites Umlenkelement
- 39: Reflektorelement
- 40: Reflektorelement
- 41: Reflektorelement
- 42: Digitale Aufnahmeeinrichtung
- 43: Versatz zwischen erstem und zweitem Umlenkelement
- 44: Einrichtung zur Einblendung einer Fixiermarke und zur direkten Beobachtung des Auges
- 45: Erster relativer Maximalwert der Konturschärfebewertung Y
- 46: Zweiter relativer Maximalwert der Konturschärfebewertung Y
- 47: Hellfeld der Testmarke

- 48: Dunkelfeld der Testmarke
- 49: Konturübergang
- 50: Digitale Bildaufnahme
- 51: Grundplatte
- A: Auge

## Patentansprüche

1. Augenrefraktometer (13) zur objektiven Brechkraftbestimmung eines Auges (A), mit einem Optometersystem zur Abbildung einer Testmarke auf die Netzhaut des Auges (A) und mit einem Beobachtungssystem (15) zur Beobachtung der auf die Netzhaut abgebildeten Testmarke (18), wobei Optometersystem und Beobachtungssystem synchron zueinander relativ zu eine Referenzstellung verstellt werden können, und wobei aus der Differenz zwischen der Einstellung, bei der die Testmarke (18) zumindest teilweise konturscharf auf die Netzhaut abgebildet wird, und der Referenzstellung ein Brechkraftparameter des Auges (A) bestimmbar ist,
**dadurch gekennzeichnet,**
**dass** am Beobachtungssystem eine digitale Aufnahmeeinrichtig (42) und eine digitale Bildverarbeitungseinheit vorgesehen ist, wobei mit der Aufnahmeeinrichtig (42) digitale Bilddaten der auf die Netzhaut abgebildeten Testmarke (18) aufgenommen werden können, und wobei die dabei erhaltenen Bilddaten in der Bildverarbeitungseinheit durch digitale Bildverarbeitung zur Feststellung des Brechkraftparameters ausgewertet werden können.

2. Augenrefraktometer nach Anspruch 1,
**dadurch gekennzeichnet,**
**dass** die digitale Aufnahmeeinrichtung (42) in der Art eines CCD-Chips ausgebildet ist.

3. Augenrefraktometer nach Anspruch 1 oder 2,
**dadurch gekennzeichnet,**
**dass** zur synchronen Verstellung von Optometersystem und Beobachtungssystem eine linear verstellbare Stelleinheit (27) vorgesehen ist, auf dem ein erstes Umlenkelement (23), das im Strahlengang (14) des Optometersystems angeordnet ist, und ein zweites Umlenkelement 38), das im Strahlengang (15) des Beobachtungssystem angeordnet ist, befestigt sind, so dass durch Verstellung der Stelleinheit (27) die Länge des Strahlengangs (14) im Optometersystem und die Länge des Strahlengangs (15) im Beobachtungssystem synchron zueinander verändert werden.

4. Augenrefraktometer nach Anspruch 3,
**dadurch gekennzeichnet,**
**dass** das erste Umlenkelement (23) und/oder das zweite Umlenkelement (38) jeweils von zwei Reflektionselementen (24, 25; 39, 40) aufweisen, die im Strahlengang (14) des Optometersystems und/oder im Strahlengang (15) des Beobachtungssystem derart angeordnet sind, dass ein zur Veränderung der Länge des Strahlengangs (14) im Optometersystem und/oder der Länge des Strahlengangs (15) im Beobachtungssystem geeigneter Posaunenzug gebildet wird.

5. Augenrefraktometer nach Anspruch 3 oder 4,
**dadurch gekennzeichnet,**
**dass** das erste Umlenkelement (23) und/oder das zweite Umlenkelement (38) auf der Stelleinheit (27) justierbar gelagert sind.

6. Augenrefraktometer nach einem der Ansprüche 3 bis 5,
**dadurch gekennzeichnet,**
**dass** das erste Umlenkelement (23) und/oder das zweite Umlenkelement (38) mit einem Versatz (43) zueinander derart auf der Stelleinheit (27) gelagert sind, dass die Länge des Strahlengans (14) im Optometersystem zwischen Testmarke (18) und Auge (A) exakt der Länge des Strahlengans (15) im Beobachtungssystem zwischen Auge (A) und digitale Aufnahmeeinrichtig (42) entspricht.

7. Augenrefraktometer nach einem der Ansprüche 3 bis 6,
**dadurch gekennzeichnet,**
**dass** die Stelleinheit (27) mit einem Servomotor (31) verstellt werden kann.

8. Augenrefraktometer nach einem der Ansprüche 3 bis 7,
**dadurch gekennzeichnet,**
**dass** die Position der Stelleinheit (27) mit einem Sensor detektiert werden kann, wobei eine Nullstellung der Stelleinheit (27) die Referenzstellung des Augenrefraktors (13) definiert, und wobei die jeweils vom Sensor gemessene Position der Stelleinheit (27) an eine Auswerteeinheit weitergeleitet und dort als aktuelle Einstellung des Augenrefraktors (13) relativ zur Referenzstellung ausgewertet werden kann.

9. Augenrefraktometer nach Anspruch 8,
**dadurch gekennzeichnet,**
**dass** die Position der Stelleinheit (27) durch Auswertung der Stellung des Servomotors (31) detektierbar ist.

10. Augenrefraktometer nach einem der Ansprüche 7 bis 9,
**dadurch gekennzeichnet,**
**dass** Servomotor (31) in der Funktion eines Schrittmotors zur Verstellung der Stelleinheit (27) in äquidistanten Schritten betrieben werden kann.

11. Augenrefraktometer nach einem der Ansprüche 1 bis 10,
**dadurch gekennzeichnet,**
**dass** als Testmarke (18) ein mittelpunktsymmetrisches Konturbild mit mehreren sich vom Mittelpunkt nach außen erstreckenden Konturübergängen (49) verwendet wird.

12. Augenrefraktometer nach einem der Ansprüche 1 bis 11,
**dadurch gekennzeichnet,**
**dass** die Testmarke (18) eine Vielzahl von abwechselnd angeordneten Hellfeldern (47) und Dunkelfeldern (48) aufweist.

13. Augenrefraktometer nach einem der Ansprüche 1 bis 12,
**dadurch gekennzeichnet,**
**dass** am Optometersystem eine LED-Beleuchtungseinrichtung (16) zur Beleuchtung der Testmarke (18) vorgesehen ist.

14. Augenrefraktometer nach einem der Ansprüche 1 bis 13,
**dadurch gekennzeichnet,**
**dass** im Strahlengang (14) des Optometersystems und/oder im Strahlengang (15) des Beobachtungssystems jeweils zumindest zwei feststehend angeordnete Linsen (21, 22; 36, 37) angeordnet sind.

15. Augenrefraktometer nach Anspruch 14,
**dadurch gekennzeichnet,**
**dass** die Linsen (21, 22; 36, 37) an einem gemeinsamen Trägerelement (20, 35) derart befestigt sind, dass die Linsen (21, 22; 36, 37) im Bereich des Posaunenzugs der Strahlengänge (14, 15) angeordnet sind.

16. Augenrefraktometer nach einem der Ansprüche 1 bis 15,
**dadurch gekennzeichnet,**
**dass** eine Einrichtung (44) zur Einblendung einer Fixiermarke ins Auge (A) vorgesehen ist.

17. Augenrefraktometer nach einem der Ansprüche 1 bis 16,
**dadurch gekennzeichnet,**
**dass** eine Einrichtung (44) zur direkten Beobachtung des Auges (A) durch einen Behandler vorgesehen ist.

18. Augenrefraktometer nach einem der Ansprüche 1 bis 17,
**dadurch gekennzeichnet,**
**dass** eine Fixationsbeleuchtung zur Fixation des Auges in einer Lage vorgesehen ist, in der mit dem Beobachtungssystem der Fundus des Auges, insbesondere der Nervenfaserkopf, aufgenommen werden kann.

19. Verfahren zum Betrieb eines Augenrefraktometer (13) zur objektiven Brechkraftbestimmung eines Auges (A) mit einem Optometersystem zur Abbildung einer Testmarke (18) auf die Netzhaut des Auges (A) und mit einem Beobachtungssystem zur Beobachtung der auf die Netzhaut des Auges (A) abgebildeten Testmarke (18), wobei Optometersystem und Beobachtungssystem synchron zueinander relativ zu eine Referenzstellung verstellt werden können, und wobei aus der Differenz zwischen der Einstellung, bei der die Testmarke (18) zumindest teilweise konturscharf auf die Netzhaut abgebildet wird, und der Referenzstellung ein Brechkraftparameter des Auges (A) bestimmbar ist,
**dadurch gekennzeichnet,**
**dass**
- bei unterschiedlichen Einstellungen des Optometersystems und Beobachtungssystem mit einer digitalen Aufnahmeeinrichtig (42) mehrer digitale Bilddatensätze aufgenommen und zusammen mit den jeweils zugeordneten Einstellungsparametern des Optometersystems und Beobachtungssystem gespeichert werden, wobei jeder einzelne Bilddatensatz die bei der jeweiligen Einstellung auf die Netzhaut abgebildeten Testmarke (18) repräsentiert,
- in einer Bildverarbeitungseinheit die Bilddatensätze durch digitale Bildverarbeitung ausgewertet werden, um jedem Bilddatensatz eine Konturschärfenbewertung zuzuordnen,
- in einer Auswerteeinheit die Konturschärfenbewertungen der Bilddatensätze ausgewertet werden, um die Einstellungen festzustellen werden, bei denen es relative Maximalwerte (45, 46) für die Konturschärfenbewertung gibt,
- in der Auswerteeinheit aus der Differenz zwischen den Einstellungen, an denen es relative Maximalwerte (45, 46) für die Konturschärfenbewertung gibt, und der Referenzstellung ein Brechkraftparameter bestimmt wird.

20. Verfahren nach Anspruch 19
**dadurch gekennzeichnet,**
**dass** in der Auswerteeinheit die Bilddatensätze mit den beiden höchsten relative Maximalwerten (45, 46) für die Konturschärfenbewertung festgestellt werden, wobei aus den entsprechend zugeordneten Einstellungen die Brechkraftparameter in den beiden Brechkrafthauptachsen abgeleitet wird.

21. Verfahren nach Anspruch 19 oder 20
**dadurch gekennzeichnet,**
**dass** als Testmarke (18) ein mittelpunktsymmetrisches Konturbild mit mehreren sich vom Mittelpunkt nach außen erstreckenden Konturübergängen (49) verwendet wird, wobei in der Bildverarbeitungseinheit ein Bilddatensatz mit dem insbesondere niedrigerem Maximalwert (46) für die Konturschärfenbewertung derart ausgewertet wird, welcher Konturübergang (49a) der Testmarke (18) mit maximaler Konturschärfe abgebildet wurden, und wobei aus der Winkeldifferenz (γ) zwischen diesem Konturübergang (49a) und der Vertikalen bzw. Horizontalen der Neigungswinkel (α) der Brechkrafthauptachsen gegenüber der Vertikalen bzw. Horizontalen abgeleitet wird.

22. Verfahren nach Anspruch 19
**dadurch gekennzeichnet,**
**dass**
- das Auge mit einer Fixationsbeleuchtung in einer Lage fixiert wird, in der mit dem Beobachtungssystem der Nervenfaserkopf, aufgenommen werden kann,
- bei unterschiedlichen Einstellungen des Beobachtungssystem mit der digitalen Aufnahmeeinrichtig mehrer digitale Bilddatensätze aufgenommen und zusammen mit den jeweils zugeordneten Einstellungsparametern des Beobachtungssystem gespeichert werden, wobei jeder einzelne Bilddatensatz den bei der jeweiligen Einstellung aufgenommenen Nervenfaserkopf repräsentiert,
- in einer Bildverarbeitungseinheit die Bilddatensätze durch digitale Bildverarbeitung ausgewertet werden, um jedem Bilddatensatz eine Konturschärfenbewertung zuzuordnen,
- in einer Auswerteeinheit die Konturschärfenbewertungen der Bilddatensätze ausgewertet werden, um die Einstellungen festzustellen werden, bei denen es relative Maximalwerte für die Konturschärfenbewertung gibt,
- in der Auswerteeinheit aus der Differenz zwischen den Einstellungen, an denen es relative Maximalwerte für die Konturschärfenbewertung gibt die Tiefe der Exkavitation des Nervenfaserkopfs bestimmt wird.
